# FASCICULE DE BREVET EUROPEEN

(11) **EP 2 842 587 B1**
(45) Date de publication et mention de la délivrance du brevet: **05.08.2020**
(21) Numéro de dépôt: 14182314.6
(22) Date de dépôt: 26.08.2014
(51) Int. Cl.: A61M 16/00, A61M 16/12, A61M 16/10, A61M 16/08

(54) **APPAREIL D'ASSISTANCE RESPIRATOIRE POUR DES PERSONNES SOUFFRANT DE TROUBLES RESPIRATOIRES**
GERÄT ZUR ATMUNGSUNTERSTÜTZUNG FÜR PERSONEN, DIE UNTER ATEMBESCHWERDEN LEIDEN
ASSISTED BREATHING DEVICE FOR PERSONS SUFFERING FROM RESPIRATORY DISORDERS

(30) Priorité: 27.08.2013 FR 1358206
(43) Date de publication de la demande: 04.03.2015
(73) Titulaire: EOVE, 64000 Pau (FR)
(72) Inventeur: COTTEAUX, Fabien, 64320 Idron (FR); JOURDAIN, Cédric, 64140 Lons (FR)
(74) Mandataire: Pittis, Olivier

(56) Documents cités:
- EP-A2- 1 621 223
- WO-A1-02/02169
- WO-A1-93/00062
- WO-A1-99/08738
- WO-A1-2008/064725
- WO-A2-2006/086190
- US-A- 4 397 306
- US-A- 5 485 850
- US-A1- 2005 098 179
- US-A1- 2007 283 958
- US-A1- 2010 078 017

## Description

### [Domaine de l'invention]

La présente invention concerne un appareil d'assistance respiratoire pour des personnes souffrant de troubles respiratoires.

### Art antérieur]

Les dispositifs de fourniture de gaz respiratoire dénommés également dispositifs d'assistance respiratoire, sont utilisés notamment dans les traitements d'affections respiratoires de patients adultes ou d'enfants. Le traitement thérapeutique de telles affections consistant à insuffler au patient de l'air à deux pressions positives par rapport à la pression ambiante, est connu depuis de nombreuses années. Ces dispositifs de fourniture de gaz respiratoires sont aptes à fournir une quantité d'air, additionnée éventuellement d'oxygène, soit sous forme d'un volume respirable quantifié soit sous la forme d'une première pression déterminée dite pression d'inspiration et une seconde pression déterminée dite pression d'expiration.

Le document WO 00/24447 concerne un dispositif de fourniture de gaz respiratoire pourvu d'un compresseur délivrant un débit de gaz sous pression à un patient au travers d'un accumulateur/silencieux aval et d'une vanne de régulation de débit. Selon ce document le surplus de gaz non nécessaire est renvoyé à un filtre/accumulateur amont au travers d'une vanne bipasse afin de limiter les pertes de gaz respiratoire.

Le document FR 2 663 547 A1 pour sa part concerne un appareil de fourniture en continu de surpression de gaz respiratoire. Cet appareil utilise une turbine haute vitesse fournissant un gaz sous une pression déterminée constante régulée par une prise de mesure de pression au niveau du masque respiratoire du patient. Cet appareil est passif en ce que la pression est maintenue en continu, les phases d'expiration étant possibles grâce à une fuite calibrée au niveau du masque respiratoire du patient.

Le document FR 2 822 384 A1 concerne un ventilateur pulmonaire mixte pourvu d'un générateur de flux, de moyens de circulation permettant de réinjecter le flux en amont du générateur lors d'une phase d'expiration d'un patient, les moyens de circulation comprenant notamment une vanne de régulation comportant deux clapets actionnés par le même actionneur permettant plusieurs modes de régulation et une optimisation des flux en fonction des phases d'inspiration et d'expiration. Selon ce document, la vitesse de rotation du générateur est maintenue constante, la pression dans le circuit patient étant contrôlée par les deux clapets qui permettent un retour de flux au générateur notamment lors des phases d'expiration.

Le document EP 1502619 B1 concerne un dispositif de fourniture de gaz respiratoire et son procédé de pilotage. Le dispositif de fourniture de gaz respiratoire décrit, a un fonctionnement qui utilise un asservissement de la turbine pour les phases inspiratoire et expiratoire et n'utilise pas de système de retour de gaz vers l'amont de la turbine, simplifiant ainsi le système, tout en permettant un fonctionnement avec circuit étanche au niveau du masque respiratoire ou du dispositif d'intubation du patient lors des phases d'inspiration et fuite lors des phases d'expirations. Pour ce faire, le dispositif de fourniture de gaz respiratoire à un patient, comporte un générateur de flux gazeux, un circuit d'amenée du flux gazeux vers un masque respiratoire ou dispositif d'intubation du patient, des moyens de mesure de pression et/ou de mesure de débit du flux gazeux, des moyens de calcul de paramètres de pression et/ou de débit et des moyens de commande de vitesse de rotation du générateur, un système de pilotage de la vitesse du générateur comportant lesdits moyens qui coopèrent afin d'asservir la vitesse de rotation de la turbine en fonction des phases d'inspiration et d'expiration en fonction de signaux de pression patient et/ou signaux de débit inspiré. Ainsi le dispositif de fourniture de gaz respiratoire est basé sur le principe de contrôle et d'asservissement d'une turbine via un clapet anti-retour associé à son clapet spontané et dont le cyclage est assuré par l'utilisation d'une vanne proportionnelle trois voies. Cette solution est limitée en performance compte tenu l'inertie de la turbine qui ne permet absolument pas une montée en pression suffisante pour satisfaire les patients essentiellement restrictifs et notamment la catégorie pédiatrique. La deuxième limite de cette solution est l'utilisation d'une vanne trois voies proportionnelle qui implique, compte tenu de sa conception, une calibration relativement précise du composant augmentant de ce fait les risques de dégradation et d'action de maintenance (recalibration), ayant un impact financier direct.

Le document EP-A-0 862 922 concerne plus particulièrement un appareil d'assistance respiratoire, du type comportant une source d'air sous pression qui fournit de l'air sous pression destiné à être acheminé, lors d'une phase inspiratoire, vers un patient au travers d'un circuit inspiratoire, et du type dans lequel de l'air expiré par le patient au cours d'une phase expiratoire est recueilli par un circuit expiratoire. Toutefois, dans un tel type d'appareil, l'expiration du patient doit se faire obligatoirement au travers du masque et il en résulte dans certains cas une gêne pour le patient, notamment en début de phase expiratoire lorsque le débit expiratoire est maximal. Cette gêne est plus particulièrement sensible lorsque la vanne expiratoire, au travers de laquelle le patient expire vers l'atmosphère, est agencée non pas directement au niveau du masque mais à une certaine distance de celui-ci. En effet, la vanne expiratoire est parfois éloignée du visage du patient pour que l'expiration ne se fasse pas directement à côté de son visage. Dans certains appareils, il est même prévu que la vanne expiratoire soit agencée au niveau de l'appareil d'assistance respiratoire. En effet, les gaz expiratoires sont alors ramenés vers l'appareil par une conduite expiratoire, séparée de la conduite inspiratoire, de sorte qu'il est possible de procéder à des analyses de débit et de pression du flux expiratoire, voire même de procéder à une analyse de la composition des gaz expirés par des capteurs qui sont alors implantés directement dans l'appareil et non plus à distance, ce qui simplifie considérablement l'intégration de ces capteurs dans un circuit de contrôle du respirateur. L'inconvénient de ce type de réalisation, dite circuit double branche, est que les pertes de charge dans le circuit expiratoire sont relativement élevées et augmentent encore l'effort que le patient doit fournir pour expirer, notamment en début d'expiration lorsque le débit expiratoire est maximum. L'appareil d'assistance respiratoire décrit dans ce document comporte des moyens permettant de réduire l'effort que doit fournir le patient lors de la phase expiratoire pour expulser les gaz expirés, notamment lors de l'utilisation d'un circuit double. L'appareil d'assistance respiratoire du type décrit précédemment, comporte à cet effet des moyens pour relier le circuit expiratoire à une source de dépression pendant au moins une partie de la phase expiratoire. Ce document EP-A-0 862 922 décrit un système complexe et onéreux du fait de la présence de quatre valves pneumatiques, qui consiste à contrôler la fourniture de gaz par le principe de deux vannes 3 voies dont l'une est disposée en série sur le circuit inspiratoire et l'autre sur le circuit expiratoire. Cette solution permet d'obtenir des performances un peu plus élevées concernant le confort du patient par rapport à la solution précédente, mais reste insatisfaisante.

On peut citer également le document WO99/08738. Dans ce document, le dispositif comporte deux ensembles de fourniture d'air reliés à un même conduit se terminant par un masque de respiration. Lorsque les deux ensembles fonctionnent, ils fonctionnent simultanément et sont en redondance permanente. En effet, la deuxième turbine vient compléter ce que fournit la première turbine dans le cas où la première turbine ne peut pas fournir suffisamment pour atteindre la ventilation requise. Le dispositif décrit a pour but de transformer deux appareils à pression d'air continue positive (CPAP) conçus pour traiter les apnées du sommeil, en un appareil plus performant visant des pathologies respiratoires plus sévères mais il ne peut en aucun cas permettre d'assurer une sécurité en cas de panne de l'un des ensembles, dans la mesure où le deuxième ensemble permet de suppléer le premier lorsque le premier n'est pas suffisant pour les besoins d'un patient. Les deux ensembles 10 et 110 ne sont pas commutables/substituables ou réversibles, les deux ensembles ne sont reliés que par un tube au masque du patient hormis l'exemple illustré par la figure 3 qui indique une connexion comportant en outre une vanne en sortie du deuxième ensemble 210, mais qui de toute façon ne permet pas à ce deuxième ensemble de fonctionner indépendamment du premier.

Le dispositif décrit dans ce document ne permet pas d'avoir une redondance intervenant en cas de panne ni une commutation/substitution automatique d'un ensemble à un autre. De plus, il ne permet pas un partage de travail entre les deux ensembles permettant d'offrir une pleine puissance des deux turbines, disponible à tout moment sans qu'il y ait à attendre un signal indiquant le manque US4397306 décrit un dispositif comportant trois ensembles de fourniture d'air reliés à un même conduit. Ce dispositif ne permet pas d' avoir une redondance en cas de panne.

### [Problème technique]

Les différents appareils d'assistance respiratoire décrits présentent un niveau de performance et de sécurité qui reste encore trop faible par rapport aux besoins. Certains patients très restrictifs, adultes comme enfants, requièrent des performances de plus en plus importantes pour palier à leur déficience ventilatoire, leur pathologie restrictive implique un niveau de pression élevée et un besoin en débit résultant très important. D'autres patients, ventilo-dépendants c'est-à-dire des patients qui nécessitent assistance 24h/24h, se doivent d'être équipé d'un appareil de secours pour prévenir tout incident provenant de l'appareil principal. Dans ce cas c'est l'entourage professionnel ou familial qui doit intervenir dans les plus brefs délais afin de procéder au changement d'appareil, ce qui implique qu'un deuxième appareil soit à disposition et prêt à être branché. En outre, cette action se déroule souvent dans la précipitation et le stress, et peut occasionner des problèmes graves durant le changement d'appareil, sans compter que durant toute cette opération le patient reste en apnée totale.

L'invention a donc pour but de remédier à au moins un des inconvénients de l'art antérieur. En particulier, l'invention vise à proposer un appareil d'assistance respiratoire qui apporte une sécurité accrue par rapport aux solutions connues tout en étant simple, fiable, à faible maintenance et peu onéreux.

En outre l'appareil proposé présente des performances relatives au débit et à la montée en pression du flux gazeux d'au moins 50% supérieures par rapport aux appareils existants qui permettent de mieux répondre notamment aux déficiences ventilatoires des patients les plus restrictifs (à savoir, les patients dont la pathologie restrictive implique un niveau de pression élevé).

A cet effet, l'invention a pour objet un appareil d'assistance respiratoire présentant des performances et une sécurité accrues par rapport aux appareils de l'état de la technique cités précédemment, ledit appareil comportant un premier ensemble de ventilation, au moins un deuxième ensemble de ventilation et un dispositif de couplage entre ces deux ensembles de sorte que le deuxième ensemble de ventilation est apte à fonctionner par substitution au premier ensemble de ventilation en cas d'arrêt du premier ensemble ou de défaillance ou de fonctionner en redondance avec le premier ensemble. Le dispositif de couplage permet de relier les circuits d'air des deux ensembles de ventilation au circuit de respiration d'un patient et en cas de défaillance de l'un des deux ensembles, une substitution automatique sur l'autre ensemble, la sécurité étant ainsi accrue. Le dispositif de couplage permet un mode de fonctionnement dit «couplage haute performance» : cas où les deux ensembles de ventilation sont mis en fonction dans l'appareil et fournissent tous les deux un flux d'air au patient, ou un mode de fonctionnement dit «couplage haute sécurité» : cas où le flux d'air est fourni par l'un des ensembles, l'autre ensemble étant en veille et apte à prendre instantanément le relais du premier en cas de détection d'anomalie de ce dernier.

### [Brève description de l'invention]

Plus particulièrement l'invention concerne un appareil d'assistance respiratoire pour une personne/patient souffrant de troubles respiratoires comprenant - un premier ensemble de ventilation, une interface de connexion pour le branchement d'un circuit de respiration du patient, ledit premier ensemble de ventilation comportant au moins un générateur d'air, au moins un capteur de pression et/ou de débit et un circuit de flux d'air relié à l'interface de connexion, - au moins un deuxième ensemble de ventilation comportant au moins un générateur d'air, au moins un capteur de pression et/ou de débit et un circuit de flux d'air relié à la dite interface de connexion ; principalement **caractérisé en ce que** l'interface de connexion de l'appareil comporte un ensemble de connexion destiné au branchement du circuit de respiration du patient et un dispositif de couplage relié aux circuits de flux d'air des ensembles de ventilation et à l'ensemble de connexion de l'appareil, le circuit de respiration du patient étant ainsi apte à être branché aux dits ensembles de ventilation via le dispositif de couplage; ledit dispositif de couplage comprenant des liaisons pneumatiques ou pneumo-électriques de couplage des ensembles de ventilation et des valves de couplage sur les liaisons de flux d'air relatives à chaque ensemble de ventilation de sorte que le deuxième ensemble de ventilation est apte à fonctionner par substitution au premier ensemble de ventilation en cas d'arrêt du premier ensemble ou de défaillance ou de fonctionner en redondance avec le premier ensemble.

Le dispositif de couplage permet d'augmenter la sécurité et permet un mode de fonctionnement assurant un couplage haute performance et/ou un couplage haute sécurité.

Ainsi, l'appareil d'assistance respiratoire comprend une interface de connexion comportant un dispositif de couplage et au moins un deuxième ensemble de ventilation et apte à fonctionner par substitution au premier ensemble de ventilation ou, simultanément avec ledit ensemble de ventilation ou, en redondance, le circuit de respiration du patient étant branché via l'interface de connexion sur l'un au moins des deux ensembles de ventilation. Le dispositif de couplage comporte des liaisons pneumatiques ou pneumo-électriques de couplage des ensembles de ventilation et des valves de couplage sur les liaisons de flux d'air relatives à chaque ensemble de ventilation assurant une sécurité accrue et permettant un mode de fonctionnement couplage haute performance et/ou couplage haute sécurité.

Chaque ensemble de ventilation comporte en outre une unité de commande de couplage apte à piloter les valves de couplage des liaisons des flux d'air relatives à chaque ensemble de ventilation afin de permettre une substitution automatique de l'un à l'autre dans les modes de fonctionnement couplage haute performance et/ou couplage haute sécurité.

**Selon d'autres caractéristiques optionnelles de l'appareil** :
- L'interface de connexion de l'appareil comporte un ensemble de connexion (301, 302) par ensemble de ventilation (100, 200), chaque ensemble de connexion étant destiné à un branchement direct d'un circuit de respiration (190, 290) sur un ensemble de ventilation. Les ensembles de ventilation ont un fonctionnement indépendant, l'un venant en secours de l'autre en cas de défaillance. Un deuxième circuit de respiration du patient peut être branché en attente sur un deuxième ensemble de ventilation,
- L'interface de connexion de l'appareil comporte un ensemble de connexion (303) destiné au branchement du circuit de respiration (190) du patient et un dispositif de couplage (300) relié aux ensembles de ventilation (100, 200) et à l'ensemble de connexion (303) de l'appareil (1), le circuit de respiration du patient étant destiné à être branché aux dits ensembles de ventilation (100, 200) via le dispositif de couplage,.
- Le dispositif de couplage comprend des liaisons pneumatiques ou pneumo-électriques de couplage des ensembles de ventilation (100, 200). Le dispositif de couplage permet un mode de fonctionnement dit «de couplage haute performance» et/ou un mode de fonctionnement «de couplage haute sécurité».
- Chaque ensemble de ventilation comporte une unité de pilotage comprenant une interface homme machine et une commande électronique apte à piloter le générateur d'air.
- Chaque ensemble de ventilation comporte des capteurs de pression et/ou de débit d'air,
- Avantageusement, le dispositif de couplage (300) comporte des valves de couplage sur les liaisons de flux d'air relatives à chaque ensemble de ventilation pour permettre un mode de fonctionnement couplage haute sécurité et chaque ensemble de ventilation comporte une unité de commande de couplage apte à piloter les valves de couplage des liaisons des flux d'air relatives à chaque ensemble de ventilation,
- Avantageusement, le dispositif de couplage comporte :
   - une première valve de couplage disposée sur la liaison du flux d'air inspiré relative au premier ensemble de ventilation et une deuxième valve de couplage disposée sur le flux d'air inspiré provenant du deuxième ensemble de ventilation,
   - une première valve de couplage disposée sur la liaison du flux d'air expiré du premier ensemble de ventilation et une deuxième valve de couplage disposée sur le flux d'air expiré relatif deuxième ensemble de ventilation

Et avantageusement, chaque ensemble de ventilation comporte une commande de couplage apte à piloter les valves de couplage.

L'invention a également pour objet un procédé de ventilation au moyen d'un appareil d'assistance respiratoire pour des personnes/patient souffrant de troubles respiratoires tel que décrit précédemment.
- Avantageusement le procédé comprend :
- La distribution d'air à une personne/patient souffrant de troubles respiratoires suivant un cycle de respiration comprenant des phases d'inspiration chacune suivie de phases d'expiration, au moyen d'au moins un des ensembles de ventilation de l'appareil d'assistance respiratoire sur lequel est branché le circuit de respiration de la personne, ladite distribution comprenant,
- en phase inspiration d'air par la personne, l'insufflation d'air au moyen d'un des ensembles de ventilation à une valeur de consigne de pression ou de débit préalablement réglée, et maintenu constant,
- en phase d'expiration d'air par la personne, une diminution par ledit ensemble de la pression ou du débit d'air jusqu'à un niveau de pression ou de débit prédéterminé (PEP ou PR).
- Avantageusement, la pression ou le débit d'air sont régulés par un asservissement de la rotation de la turbine du générateur d'air, réalisé par l'unité de pilotage à partir de données de mesure des capteurs de pression et/ou de débit de l'un ou des deux ensembles de ventilation,
- Selon un premier mode, les phases d'inspiration et d'expiration sont mises en oeuvre au moyen d'un premier ensemble de ventilation, un deuxième ensemble de ventilation étant substitué au premier pour la mise en oeuvre de nouvelles phases d'inspiration et d'expiration, le premier ensemble de ventilation étant arrêté ou défaillant.
- Selon un deuxième mode : - la phase d'inspiration comprend la fourniture d'air simultanément par un premier et un deuxième ensemble de ventilation, l'air étant combiné par le dispositif de couplage des ensembles de ventilation ; - la phase d'expiration comprend une diminution de la pression ou débit fournis par les ensembles de ventilation et le vidage du ballonnet de la vanne d'expiration du circuit du patient. Ce mode de fonctionnement correspond à un mode de couplage haute performance qui permet de coupler les performances de deux ensembles de ventilation, les deux ensembles de ventilation fonctionnent et fournissent en même temps le flux d'air en phase d'inspiration ce qui permet d'atteindre le niveau de pression ou débit désiré plus rapidement, et ce qui permet de vider plus rapidement le ballonnet de la valve d'expiration pendant la phase d'expiration et ainsi d'améliorer l'effort expiratoire du patient:
   Selon un troisième mode, la phase d'inspiration et d'expiration sont mises en oeuvre au moyen d'un premier ensemble de ventilation, un deuxième ensemble de ventilation est en veille et reçoit par le dispositif de couplage des informations de mesure de pression et/ou de débit relatives au premier ensemble de ventilation en action et un signal de synchronisation pour le pilotage de la turbine du générateur d'air afin de poursuivre une phase d'inspiration et/ou d'expiration en cours lorsqu'un signal de défaillance de l'un des deux ensembles est reçu (notamment informations de débit et/ ou de pression et/ou d'alimentation électrique anormales.)

Pour ces trois modes de mise en œuvre des phases d'inspiration et d'expiration, les ensembles de ventilation peuvent être pilotés au moyen de mesures de pression (mode pression) ou au moyen de mesures de débit (mode volume).

D'autres particularités et avantages de l'invention apparaitront à la lecture de la description suivante faite à titre d'exemple illustratif et non limitatif, en référence aux Figures annexées qui représentent :
La figure 1, un schéma d'un appareil suivant un premier exemple de réalisation,
La figure 2, un schéma d'un appareil selon la présente invention suivant un deuxième exemple de réalisation.

Le terme «air» désigne de l'air ou de l'air enrichi en oxygène.

Le terme « patient » concerne une personne nécessitant une assistance respiratoire et susceptible d'être branché sur l'appareil objet de l'invention.

Le terme « opérateur » désigne une personne, personnel médical ou patient lui-même ou personne de l'entourage du patient apte à effectuer les réglages et/ou paramétrages de l'appareil à sa mise en fonction.

Le terme de « valeur de pression proximale » désigne la valeur de la pression mesurée au plus proche du patient.

Le terme de « valeur de débit proximal » désigne la valeur du débit mesuré au plus proche du patient.

Le terme de « valeur de pression sécurité » désigne la valeur de la pression mesurée au plus proche de la sortie du générateur d'air.

Le terme de « valeur de débit interne » désigne la valeur du débit mesuré au plus proche du générateur d'air.

Dans la suite on désigne par « mode pression » le pilotage des ensembles de ventilation au moyen de mesures de pression et par « mode volume » le pilotage des ensembles de ventilation au moyen de mesures de débit.

Dans la suite on désigne « circuit respiratoire du patient » un circuit pneumatique destiné à être branché sur l'appareil d'assistance respiratoire pour insuffler de l'air au patient. Ce circuit peut comporter, mais pas obligatoirement, une valve d'expiration.

### [Description détaillée de l'appareil].

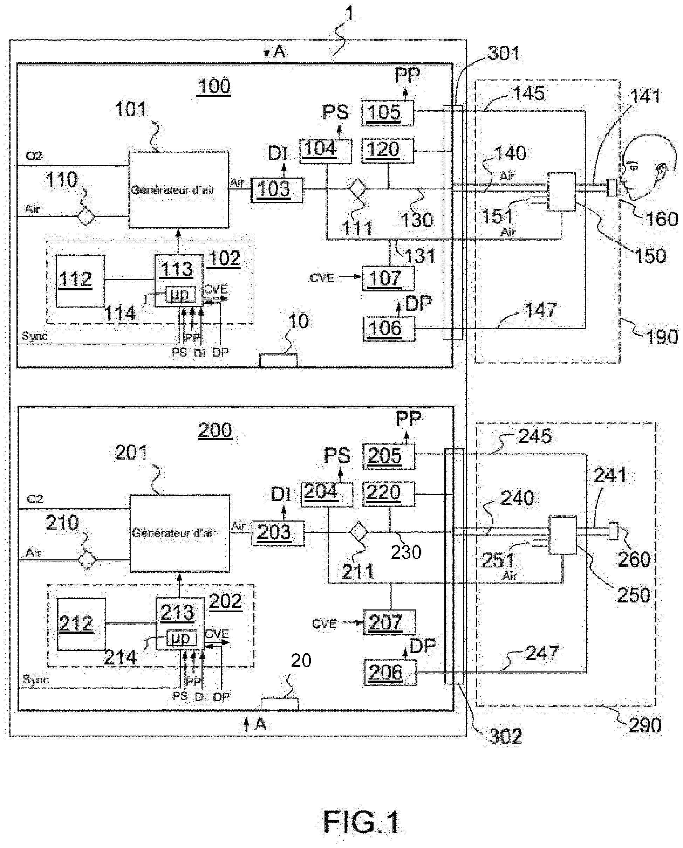

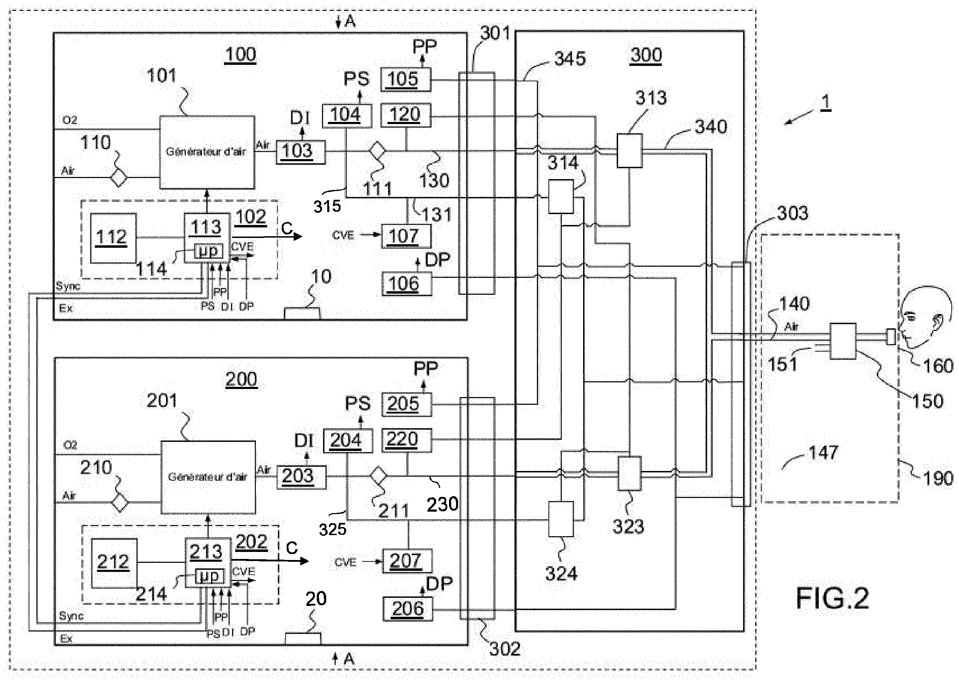

Dans les exemples illustrés par les figures 1 et 2, l'appareil d'assistance respiratoire 1 comporte deux ensembles de ventilation 100, 200. Bien entendu l'appareil peut comporter trois ensembles de ventilation ou plus selon les applications prévues. Dans le cas d'appareils destinés à des services médicaux (hôpitaux par exemple), il peut être prévu que l'appareil comporte plus de deux ensembles de ventilation et ainsi permettre d'apporter une assistance respiratoire à plusieurs patients en même temps tout en réservant au moins un ensemble de ventilation de secours dans l'appareil.

De préférence l'appareil 1 se présente sous forme d'un boitier enfermant les ensembles de ventilation. Chaque ensemble de ventilation 100, 200 peut se présenter sous forme d'un bloc de ventilation amovible indépendant des autres blocs de ventilation et peut ainsi être retiré de l'appareil aisément. Cette organisation en blocs de ventilation amovibles permet de réaliser un remplacement rapide au sein de l'appareil en cas de panne, facilitant ainsi la maintenance de l'appareil, et la mobilité éventuelle du patient puisque l'appareil est électriquement et pneumatiquement autonome.

L'appareil 1 comprend en outre une interface de connexion munie d'au moins un ensemble de connexion 301, 302 par ensemble de ventilation 100, 200.

Chaque ensemble de connexion 301, 302 est destiné à un branchement direct ou indirect d'un circuit de respiration 190, 290, sur un ensemble de ventilation.

L'interface de connexion pour le branchement d'un circuit de respiration, selon l'exemple de réalisation illustré par la figure 2, comporte un dispositif de couplage 300 et un ensemble de connexion 303.

Le pilotage en mode pression est réalisé par les capteurs de pression sécurité 104 et capteur de pression proximale 105 correspondant à l'ensemble 100 et aux capteurs de pression sécurité 204 et capteur proximale 205 pour l'ensemble de ventilation 200, les signaux de mesures envoyés par ces capteurs étant pris en compte comme cela va être détaillé dans la suite pour les premier, deuxième et troisième modes de fonctionnement lors des phases d'inspiration et d'expiration.

Le pilotage en mode débit est réalisé par des capteurs de débit interne 103 et de débit proximal 106 pour l'ensemble de ventilation 100 et par les capteurs de débit interne 203 et par le capteur de débit proximal 206 pour l'ensemble de ventilation 200.

L'appareil d'assistance respiratoire 1 permet la propulsion d'air (parfois enrichi en oxygène) dans les poumons d'un patient, adulte ou enfant atteint d'insuffisance respiratoire chronique ou aigüe, suivant des modes de ventilation préétablis et relatifs à leurs pathologies.

Le patient possède un circuit de respiration 190 comportant une valve d'expiration 150, munie d'un passage d'expiration 151, reliée par un tuyau 141 à une terminaison destinée à fournir de l'air au patient. Cette terminaison peut être constituée d'un masque ou d'une canule 160 pour les patients trachéotomisés. Le circuit de respiration 190 du patient est branché à l'appareil par une liaison pneumatique (un tuyau par exemple) d'alimentation en air 140, par une liaison pneumatique 145 permettant une mesure de pression proximale et par une liaison pneumatique 147 permettant une mesure de débit proximal.

Dans l'exemple de réalisation illustré sur la figure 1, un deuxième circuit de respiration 290 de secours peut être prévu. Ce circuit comporte les mêmes éléments que le premier à savoir une valve d'expiration 250, munie d'un passage d'expiration 251, reliée par un tuyau 241 à une terminaison destinée à fournir de l'air au patient. Cette terminaison peut être constituée d'un masque ou d'une canule 260 pour les patients trachéotomisés. Le circuit de respiration du patient est branché à l'appareil par une liaison pneumatique (un tuyau par exemple) d'alimentation en air 240, par une liaison pneumatique 245 permettant une mesure de pression proximale et par une liaison pneumatique 247 permettant une mesure de débit proximal.

Avantageusement les deux ensembles de ventilation 100, 200 comportent les mêmes éléments de manière à permettre d'avoir une autonomie pour chaque ensemble et de préférence une redondance complète. Ainsi, de préférence les deux ensembles de ventilation ont une autonomie électrique et pneumatique, chacun ayant une alimentation électrique autonome comprenant une alimentation secteur A et une batterie 10 et 20, et chacun ayant un générateur d'air 101 et 201 de type turbine ou compresseur ou soufflet. Cette redondance permet d'augmenter la sécurité et d'offrir les différents modes de fonctionnement décrit précédemment.

Sur la figure 1, l'appareil 1 comporte deux ensembles de ventilation 100 et 200. Chaque ensemble de ventilation comporte un ensemble de connexion 301, 302. Deux circuits de respiration du patient 190 et 290 sont branchés à l'appareil 1. Un circuit de respiration 190 est branché à l'ensemble de ventilation 100 au moyen du premier ensemble de connexion 301 et un deuxième circuit de respiration 290 est branché à l'autre ensemble de ventilation 200 au moyen du deuxième ensemble de connexion 302.

Sur la figure 2, l'appareil 1 comporte deux ensembles de ventilation 100 et 200 Un circuit de respiration du patient 190 est branché sur l'appareil 1 et indirectement aux deux ensembles de ventilation. Ce branchement est réalisé au moyen de l'interface de connexion qui comporte dans ce cas, l'ensemble de connexion 303 à l'appareil et un dispositif de couplage 300 connecté aux deux ensembles de ventilation. Avantageusement, le dispositif de couplage 300 peut se présenter sous forme d'un module amovible comprenant l'ensemble de connexion 303 destiné au branchement du circuit de respiration. Ledit module est apte à être connecté sur les ensembles de ventilation de l'appareil 1.

Le dispositif de couplage 300 comprend des liaisons pneumatiques ou pneumo-électriques de couplage des deux ensembles de ventilation. Le dispositif de couplage permet un mode de fonctionnement dit «de couplage haute performance» et/ou un mode de fonctionnement «de couplage haute sécurité».

Le premier ensemble de ventilation 100 comprend le générateur d'air 101, une unité de pilotage 102, les capteurs de pression et de débit, une commande de valve d'expiration 107 (non nécessaire lorsque le circuit de respiration du patient ne comporte pas de valve d'expiration) et avantageusement une commande de couplage 120 pilotée par un signal C généré par l'unité de pilotage 102. La commande de couplage comporte avantageusement une valve dont l'ouverture et/ou la fermeture sont commandées par le signal C. La commande de couplage 120 n'est pas nécessaire ou pas utilisée dans le premier mode de fonctionnement. Cette commande de couplage n'est nécessaire que lorsque les deux ensembles sont couplés afin de permettre une substitution automatique de l'un à l'autre selon les deuxièmes et troisièmes modes de fonctionnement. En outre, de préférence le premier ensemble comporte une alimentation électrique autonome 10 (batterie par exemple). Le générateur d'air 101 est avantageusement du type micro-turbine, ladite micro-turbine est pilotée par une commande électronique CDV qui permet de réguler sa vitesse de rotation. Cette régulation est réalisée en fonction du besoin en pression ou en débit, exprimé par le mode de ventilation sélectionné par l'opérateur à partir de l'interface 112 et calculé par le module de calcul 114 de la commande électronique 113, qui reçoit à cette fin, les signaux de mesure des capteurs de pression 104, 105, pression sécurité et pression proximale et/ou des capteurs de débit 103, 106, débit interne et débit proximal. L'interface homme machine 112 comporte avantageusement un clavier et un écran reliés à la commande électronique 113 permettant à l'opérateur de rentrer le mode de fonctionnement et les valeurs de pression et/ou débit de consigne.

Le deuxième ensemble de ventilation 200 sert d'ensemble de secours et comprend les mêmes éléments que le premier ensemble 100, le nombre de capteurs et de commandes, leur position ainsi que leur fonctionnement, sont avantageusement les mêmes. Les repères des éléments du deuxième ensemble 200 sont les mêmes que ceux du premier ensemble mais commencent par un 2 : générateur 201, capteur de débit interne 203, capteur de débit proximal 206, capteur de pression sécurité 204, capteur de pression proximale 205, commande de la valve d'expiration 207, l'unité de pilotage 202, l'interface homme machine 212, la commande électronique 213, le module de calcul 214 et la commande de couplage 220. Tout comme la commande de couplage 120, la commande de couplage 220 comporte une valve. Cette valve est pilotée par le signal généré par l'unité 202.

Dans les exemples donnés, le circuit de respiration du patient comporte une valve d'expiration 150. Chaque ensemble comporte une commande de valve d'expiration 107, 207. Cette commande 107, 207 est pilotée par un signal CVE généré par l'unité 102 et ou 202. La commande de valve d'expiration 107, 207 comporte une vanne qui, lorsqu'elle est ouverte, permet de vider le ballonnet de la valve d'expiration 150.

Le cyclage de la respiration se décompose pour l'ensemble de ventilation par une action d'accélération, de stabilisation et de freinage. Ce cyclage est directement assuré par la turbine du générateur d'air 101 sur pilotage de l'unité de pilotage 113, ainsi la turbine définit le cycle respiratoire du patient au moyen des valeurs de fréquence et de rapport cyclique préalablement réglées par l'opérateur. Le cyclage de la respiration du patient comporte une phase d'inspiration suivie d'une phase d'expiration.

Les capteurs de pression 104, 105 (pression sécurité et pression proximale) permettent d'asservir, ou de limiter, la vitesse de rotation de la turbine en fonction du besoin en pression exprimé par le réglage du mode de ventilation en cours. La pression proximale est la valeur de pression de référence, elle détermine la valeur de pression la plus proche du patient, et donc la plus efficace. La pression de sécurité est principalement utilisée lors de l'absence ou de la défaillance de la mesure de pression proximale. Cette valeur de pression peut également servir à vérifier le bon fonctionnement de la pression proximale et traquer un éventuel disfonctionnement.

Les capteurs de débit 103, 106 (débit interne et débit proximal) permettent d'asservir la rotation de la turbine en fonction du besoin en débit exprimé par le réglage du mode de ventilation en cours. Le capteur de débit proximal servant principalement à mesurer le volume d'air administré aux patients en mesurant son débit expiré, permis par la position du capteur 106 situé en amont de la valve d'expiration 150 par rapport au patient.

La commande 107 de la valve d'expiration comprend une vanne 2 voies. En phase d'expiration, elle permet de vider le ballonnet (non représenté) de la valve d'expiration 150 située sur le circuit 190 du patient, et d'autoriser l'expiration du patient via le passage d'expiration 151. Cette valve 150 est fermée en phase d'inspiration et permet de regonfler le ballonnet (qui obture alors le passage d'expiration 151) grâce à la pression résiduelle maintenue par le générateur d'air 101 resté à un régime minimum, et autorise ainsi au flux d'air d'être redirigé vers le patient et de réaliser une insufflation.

L'interface homme machine 112 est principalement constituée d'un écran et d'un clavier (non représentés) permettant le réglage des différentes valeurs nécessaires à la mise en œuvre des cycles respiratoires notamment : pression, débit, fréquence de fonctionnement de la turbine du générateur d'air.

De préférence, des filtres antibactériens 110, 210, 111 et 211 sont prévus afin d'isoler chaque ensemble de ventilation, en entrée et sortie, des problèmes de contamination. Ces filtres peuvent être disposés à l'intérieur ou à l'extérieur de l'appareil en fonction du besoin.

### Procédé de ventilation au moyen de l'appareil d'assistance 1 selon un premier mode de ventilation.

On entend par « premier mode de ventilation » le cas où un seul ensemble d'assistance respiratoire est mis en fonction dans l'appareil et fournit le flux d'air au patient, le circuit de respiration du patient, muni de la valve d'expiration, est branché sur cet ensemble soit directement au moyen de l'interface de connexion de l'appareil soit indirectement via le dispositif de couplage, ledit ensemble de ventilation est désigné par la suite « ensemble principal ».

Les paramètres du mode de ventilation sont réglés grâce au clavier et à l'afficheur de l'appareil.

En phase inspiration du mode pression : la commande 107 de la valve d'expiration 150 se ferme pour permettre de gonfler le ballonnet de ladite valve d'expiration. Cette commande est réalisée par une vanne 107 pilotée par un signal de commande CVE généré par la commande numérique 113. Le générateur d'air 101 est piloté et régulé par la commande numérique 113 à la valeur de la consigne de pression inspiratoire sélectionnée par l'opérateur, en se basant sur la mesure PS du capteur de pression sécurité 104 ou bien de la mesure de pression proximale PP 105, dans le cas où la connexion au capteur 105 a été faite, la connexion se faisant au moyen de la liaison 145.

Ce niveau de pression sera maintenu constant jusqu'à la fin de la phase d'inspiration qui peut être déterminé par un temps (réglage de fréquence / rapport cyclique) ou bien par un niveau de débit inspiré appelé trigger expiratoire.

En phase expiration du mode pression : le générateur d'air 101 engage une phase de freinage, cette phase est pilotée par la commande 113 de façon progressive afin de protéger la turbine de tout endommagement. La pression dans le circuit 190 baisse considérablement. Cette baisse importante de pression, combinée à l'ouverture de la vanne de la commande 107, permet au ballonnet situé sur la valve d'expiration 150, de se vider au travers de ladite vanne. Une fois le ballonnet dégonflé le patient peut très facilement expiré par le passage 151 prévu à cet effet.

Lorsque la phase de freinage est terminée, le générateur d'air 101 est mis en mode de régulation de pression et permet une régulation à la valeur PEP de pression expiratoire positive sélectionnée par l'opérateur. Cette pression PEP est généralement plus basse (entre 2 et 4 mbar minimum) que la pression d'inspiration, mais peut également être désactivée. Dans ce cas précis, la turbine est pilotée à une vitesse relativement faible qui permet de fournir un léger débit c'est-à-dire un débit insuffisant pour créer une pression significative PR mais qui permet de rincer le tuyau 140 d'éventuel gaz d'expiration,

Ce niveau de pression significative PR (ou de débit de rinçage) est maintenu constant jusqu'à la fin de la phase d'expiration qui peut être déterminé par un temps (réglage de fréquence / rapport cyclique) ou bien par la détection d'une inspiration du patient (également dénommé par « trigger inspiratoire ») qui est généralement obtenue à partir d'une variation de la pression sécurité ou de pression proximale et/ou du débit interne ou du débit proximal.

En phase inspiration du mode volume : la commande de valve d'expiration 107 se ferme pour permettre de gonfler le ballonnet de la valve d'expiration 150. Le générateur d'air 101 est piloté et régulé par la commande électronique 113 à la valeur de la consigne de débit sélectionné par l'opérateur, et à partir de la mesure du capteur de débit. Ledit niveau de débit est contrôlé suivant le profil de débit déterminé par les réglages du mode en cours jusqu'à la fin de la phase d'inspiration qui peut être déterminé par un temps (réglage de fréquence / rapport cyclique) ou bien par un niveau de débit inspiré appelé trigger expiratoire.

En phase expiration du mode volume : le générateur d'air 101 engage une phase de freinage pilotée par la commande électronique 113 de façon progressive afin de protéger la turbine de tout endommagement, la pression dans le circuit 190 du patient baisse considérablement. Cette baisse importante de pression, combinée à l'ouverture de la vanne 107, permet au ballonnet situé sur la valve d'expiration 150, de se vider au travers de la commande 107 (de la vanne de cette commande). Une fois le ballonnet dégonflé le patient peut très facilement expirer par le passage 151 prévu à cet effet.

Une fois la phase de freinage terminée, le générateur d'air 101 est remis en mode de régulation de pression et régule à la valeur PEP de pression expiratoire positive sélectionnée par l'utilisateur. Cette pression PEP est généralement bien plus basse que la pression d'inspiration [exemples de valeurs comparatives), mais peut également être désactivée. Dans ce cas précis, la turbine est pilotée à une vitesse relativement faible mais qui permet de fournir un léger débit c'est-à-dire un débit insuffisant pour créer une pression significative mais qui permet de rincer le tuyau 140 d'éventuel gaz d'expiration. Ledit niveau de pression (ou de débit de rinçage) est maintenu constant jusqu'à la fin de la phase d'expiration qui peut être déterminé par un temps (réglage de fréquence / rapport cyclique) ou bien par le déclenchement du trigger inspiratoire généralement calculé sur une variation de la pression sécurité ou pression proximale et/ou du débit interne ou du débit proximal.

### Procédé de ventilation au moyen de l'appareil d'assistance 1 selon un deuxième mode de ventilation dit « couplage haute performance ».

On entend par deuxième mode de ventilation, le cas où les deux ensembles d'assistance respiratoire sont mis en fonction dans l'appareil 1 et fournissent tous les deux un flux d'air au patient, le circuit de respiration 190 du patient muni de la valve d'expiration 150 est branché à l'appareil. Le branchement aux deux ensembles de ventilation est réalisé de façon indirecte au moyen du circuit de couplage 300.

Le principe du couplage haute performance consiste à coupler les performances des deux ensembles de ventilation et de combiner leur fonctionnement afin de pouvoir bénéficier de la puissance des deux générateurs d'air 101 et 201. Les deux ensembles fonctionnent simultanément, l'un des ensembles est l'ensemble maitre (principal) pour la synchronisation des phases du cycle de respiration et l'autre l'ensemble esclave (secours).

Les phases d'inspiration et d'expiration, que les mesures de pression (mode pression) ou de débit (mode débit) soient utilisées, se déroulent exactement de la même manière que décrit précédemment, sauf à avoir un des deux ensembles maitre et l'autre esclave. Les deux ensembles de ventilation 100, 200 utilisent leurs références de pression sécurité et de pression proximale respectives, mais c'est l'ensemble de ventilation maître qui coordonne et synchronise les cycles de respiration. La synchronisation est réalisée par une liaison électrique qui fournit un signal SYNC de synchronisation entre les deux unités de pilotage 102 et 202.

Après réglage des paramètres de fonctionnement à savoir pression de consigne et/ou débit de consigne sur un des deux ensembles de ventilation pris comme ensemble maître, les deux ensembles s'activent en même temps pour atteindre les niveaux de pression ou débit désirés. Par exemple, si une pression de 25mbar doit être atteinte en phase d'inspiration, les deux ensembles vont débiter de l'air en même temps afin d'atteindre cet objectif au plus vite - contrôlé par les différents capteurs de pression, capteurs de pression sécurité 104, 204 et capteurs de pression proximale 105, 205. Un asservissement est mis en œuvre par les commandes électroniques 113 et 213 de façon à éviter toute instabilité de l'appareil due à la présence d'action des deux ensembles de ventilation sur la même liaison 140. La phase d'expiration est menée de la même façon et bénéficie en plus de la double performance des deux commandes 107 et 207 de la valve d'expiration 150 qui constituent la commande de ladite valve d'expiration. Le ballonnet de la valve d'expiration est vidé deux fois plus vite via les deux commandes de valve 107 et 207 et regonflé deux fois plus vite au moyen des deux générateurs d'air 101 et 201.

Ce mode de fonctionnement est notamment rendu possible par l'interconnexion des éléments suivants et de la manière suivante :

Connexion des capteurs de pression sécurité 104 et 204 des deux ensembles 100 et 200,

Connexion des capteurs de pression proximale 105 et 205 des deux ensembles 100 et 200,

Connexion des capteurs de débit interne 103 et 203 des deux ensembles 100 et 200,

Connexion des capteurs de débit proximal 106 et 206 des deux ensembles 100 et 200.

Le dispositif de couplage 300 comporte des liaisons permettant de réaliser ces connexions et est connecté à l'ensemble formé par les deux ensembles de ventilation. Avantageusement le dispositif de couplage 300 est amovible. Le dispositif 300 se branche sur les interfaces de connexion 301 et 302 de chaque ensemble de ventilation 100 et 200.

### Procédé de ventilation au moyen de l'appareil d'assistance 1 selon un troisième mode de ventilation dit « couplage haute sécurité ».

Le procédé consiste à fournir le flux d'air par un des ensembles, l'autre appareil étant en veille et apte à prendre instantanément le relais du premier en cas de détection d'anomalie de ce dernier. Pour cela, on met en œuvre un couplage de haute sécurité consistant à coupler les deux ensembles de ventilation 100 et 200 de l'appareil 1 formant l'un, un ensemble de ventilation principal et l'autre, un ensemble de ventilation de secours en veille c'est-à-dire en fonctionnement et en attente d'une commande pour poursuivre le cycle commencé. Ce mode permet ainsi de réaliser une version hautement sécurisée de l'appareil par un principe de redondance totale.

Les deux ensembles de ventilation sont parfaitement similaires et autonomes et possèdent les mêmes réglages de fonctionnement. Ils possèdent tous deux un générateur d'air 101, 201, l'unité de pilotage 102, 202 et les éléments de mesure et de contrôle associés. Afin de palier à tout défaut d'alimentation, deux alimentations de type batterie 10, 20 sont également prévues.

L'un des ensembles de ventilation constitue l'ensemble principal c'est-à-dire celui qui assure seul la ventilation du patient selon le fonctionnement normal décrit ci-dessus. Cet ensemble est couplé au deuxième ensemble de ventilation qui assure la fonction ensemble de secours via le couplage pneumatiques ou pneumo-électriques des deux ensembles et au signal de « vie » à savoir un signal d'horloge SYNC du premier ensemble reçu par le deuxième ensemble.

L'ensemble de ventilation secours est en veille, et contrôle en permanence la réception du signal de vie SYNC et les signaux de pression et de débit à sa disposition. Si l'un ou l'autre des signaux indique une valeur anormale, ou lorsque le signal de vie SYNC n'est pas reçu, l'ensemble de ventilation secours envoie un signal d'extinction EX au ensemble de ventilation principal et prend instantanément le relais. Une alarme de basculement est émise afin d'avertir le patient et son entourage de la déficience de l'ensemble principal. L'ensemble secours ayant été préalablement paramétré comme l'ensemble principal, le fonctionnement de la ventilation se poursuit exactement de la même façon. Le patient n'a pas à être débranché et aucune action n'est nécessaire par l'entourage, hormis le changement ultérieur de l'ensemble principal défaillant.

Ce mode de fonctionnement est notamment rendu possible par l'interconnexion des éléments suivants et de la manière suivante :
Connexion capteurs de pression sécurité 104, 204 des deux ensembles
Connexion capteurs de pression proximale 105, 205 des deux ensembles
Connexion capteurs de débit interne 103, 203 des deux ensembles avec les valves de couplage d'inspiration 313, 323 pilotées respectivement par l'ensemble opposé, la valve 313 est pilotée par l'ensemble 200 et la valve 323 par l'ensemble 100.
Connexion capteurs de débit proximal 106, 206 des deux Ensembles 100 et 200,
Connexion des commandes de valve d'expiration des deux ensembles avec les valves de couplage d'expiration 314, 324, pilotées respectivement par l'ensemble opposé, la valve 314 étant pilotée par l'ensemble 200 et la valve 324 par l'ensemble 100.

Le dispositif de couplage 300 comporte les éléments qui permettent de mettre en œuvre ce fonctionnement. Les valves de couplage d'expiration 314 et 324 permettent d'éviter le dégonflage du ballonnet de la valve d'expiration 150 via le générateur d'air de l'ensemble de ventilation qui est en veille lorsque deux ensembles sont ainsi interconnectés. Les deux valves de couplages sont connectées directement par les liaisons respectivement 315 et 325 aux sorties des générateurs d'air 101 et 210.

Les deux valves deux voies de couplage d'inspiration 313, 323 du dispositif de couplage permettent de bloquer respectivement la sortie principale de l'ensemble principal lorsque l'ensemble secours ventile et la sortie principale de l'ensemble secours lorsque l'ensemble principal ventile. Les deux commandes 120 et 220 des valves d'inspiration sont connectés directement aux sorties des générateurs d'air.

Dans l'exemple de réalisation illustré par la figure 1, la substitution d'un ensemble de ventilation par au moins un deuxième ensemble de ventilation est manuelle, et se réduit à brancher sur les ensembles de connexion 301 ou 302, le circuit de respiration 190 de la personne et à démarrer le générateur d'air, l'appareil restant alimenté électriquement dès qu'un ensemble de ventilation est utilisé. De préférence un deuxième circuit de respiration 290 peut être prévu et branché en attente sur l'ensemble inactif. Ainsi, en cas d'arrêt ou de défaillance de l'ensemble de ventilation mis en fonction (actif), le deuxième ensemble de ventilation de l'appareil est mis en fonction par une personne ou le patient lui-même sans qu'il soit nécessaire de réaliser des branchements complexes.

Dans l'exemple de réalisation illustré par la figure 2, les modes de fonctionnements dit « couplage haute performance » ou couplage « haute sécurité » peuvent être mis en œuvre. Le circuit de respiration 190 du patient est branché sur l'appareil 1 via le dispositif de couplage 300 et, est par conséquent, connecté indirectement à l'un des ensembles ou aux deux ensembles selon le mode de fonctionnement choisi.

## Revendications

1. Appareil d'assistance respiratoire (1) pour une personne/patient souffrant de troubles respiratoires comprenant : - un premier ensemble de ventilation (100), une interface de connexion pour le branchement d'un circuit de respiration (190) du patient, ledit premier ensemble de ventilation comportant au moins un générateur d'air (101), au moins un capteur de pression et/ou de débit et un circuit de flux d'air relié à l'interface de connexion, - au moins un deuxième ensemble de ventilation (200) comportant au moins un générateur d'air, au moins un capteur de pression et/ou de débit et un circuit de flux d'air relié à la dite interface de connexion ; **caractérisé en ce que** l'interface de connexion de l'appareil comporte un ensemble de connexion (303) destiné au branchement du circuit de respiration (190) du patient et un dispositif de couplage (300) relié aux circuits de flux d'air des ensembles de ventilation (100, 200) et à l'ensemble de connexion (303) de l'appareil (1), le circuit de respiration du patient étant ainsi apte à être branché aux dits ensembles de ventilation (100, 200) via le dispositif de couplage (300); ledit dispositif de couplage (300) comprenant des liaisons pneumatiques ou pneumo-électriques de couplage des ensembles de ventilation (100, 200) et des valves de couplage (313, 314 ; 323, 324) sur les liaisons de flux d'air relatives à chaque ensemble de ventilation, chaque ensemble de ventilation comportant en outre une commande de couplage (120, 220) apte à piloter les valves de couplage des liaisons des flux d'air relatives à chaque ensemble de ventilation afin de permettre une substitution automatique de l'un à l'autre dans les modes de fonctionnement :
- couplage haute performance, où les deux ensembles de ventilation sont mis en fonction dans l'appareil et fournissent tous les deux un flux d'air au patient, et/ou
- couplage haute sécurité, où le flux d'air est fourni par l'un des ensembles, l'autre ensemble étant en veille et apte prendre instantanément le relais du premier en cas de détection d'anomalie de ce dernier.

2. Appareil d'assistance respiratoire (1) pour une personne/patient souffrant de troubles respiratoires selon la revendication 1, caractérisé en ce chaque ensemble de ventilation comporte une unité de pilotage (102, 202) apte à piloter les valves de couplage des liaisons des flux d'air relatives à chaque ensemble de ventilation.

3. Appareil d'assistance respiratoire selon la revendication 1, **caractérisé en ce que** l'unité de pilotage (102, 202) comprend une interface homme machine et une commande électronique (113, 213) apte à piloter le générateur d'air.

4. Appareil d'assistance respiratoire (1) pour une personne/patient souffrant de troubles respiratoires selon la revendication la revendication 2, **caractérisé en ce que** la commande de couplage comporte une valve dont l'ouverture et/ou la fermeture sont commandées par un signal généré par l'unité de pilotage (102, 202) de manière à permettre la substitution automatique d'un ensemble de ventilation à l'autre selon les modes de fonctionnement couplage haute performance et/ou couplage haute sécurité.

5. Appareil d'assistance respiratoire selon l'une quelconque des revendications précédentes, **caractérisé en ce que** chaque ensemble de ventilation comporte des capteurs de pression et/ou de débit d'air.

6. Appareil d'assistance respiratoire selon la revendication 1, caractérisé en ce le dispositif de couplage (300) comprend:
- une première valve de couplage (313) disposée sur la liaison du flux d'air inspiré relative au premier ensemble de ventilation et une deuxième valve de couplage (323) disposée sur le flux d'air inspiré provenant du deuxième ensemble de ventilation (100),
- une première valve de couplage (314) disposée sur la liaison du flux d'air expiré du premier ensemble de ventilation et une deuxième valve de couplage (324) disposée sur le flux d'air expiré relatif deuxième ensemble de ventilation (200),
- la commande de couplage (120, 220) de chaque ensemble de ventilation étant apte à piloter lesdites valves de couplage (313,314 ; 323,324).

7. Appareil d'assistance respiratoire selon l'une quelconque des revendications précédentes, **caractérisé en ce que**, le dispositif de couplage (300) se présente sous forme d'un module amovible comprenant l'ensemble de connexion (303) destiné au branchement du circuit de respiration.

8. Appareil d'assistance respiratoire selon l'une quelconque des revendications précédentes, **caractérisé en ce que** chaque ensemble de ventilation (100, 200) comprend un capteur de pression de sécurité (104, 204), un capteur de pression proximale (105, 205), un capteur de débit interne (103, 203), un capteur de débit proximal (106, 206) et **en ce que** le dispositif de couplage (300) assure l'interconnexion des deux ensembles de ventilation en reliant lesdits éléments suivants :
- Connexion des capteurs de pression sécurité (104 et 204) des deux ensembles (100 et 200),
- Connexion des capteurs de pression proximale (105 et 205) des deux ensembles (100 et 200),
- Connexion des capteurs de débit interne (103 et 203) des deux ensembles (100 et 200),
- Connexion des capteurs de débit proximal (106 et 206) des deux ensembles (100 et 200).

9. Appareil d'assistance respiratoire selon l'une quelconque des revendications précédentes, **caractérisé en ce que** chaque ensemble de ventilation (100, 200) se présente sous la forme d'un bloc de ventilation amovible indépendant des autres blocs de ventilation de l'appareil.

## Patentansprüche

1. Gerät zur Atmungsunterstützung (1) für eine Person/einen Patienten, die/der unter Atembeschwerden leidet, umfassend: - eine erste Beatmungsanordnung (100), eine Verbindungsschnittstelle zum Anschluss eines Atemkreislaufs (190) des Patienten, wobei die erste Beatmungsanordnung mindestens einen Luftgenerator (101), mindestens einen Druck- und/oder Durchflusssensor und einen mit der Verbindungsschnittstelle verbundenen Luftstromkreislauf aufweist, - mindestens eine zweite Beatmungsanordnung (200), die mindestens einen Luftgenerator, mindestens einen Druck- und/oder Durchflusssensor und einen mit der Verbindungsschnittstelle verbundenen Luftstromkreislauf aufweist; **dadurch gekennzeichnet, dass** die Verbindungsschnittstelle des Geräts eine Verbindungsanordnung (303) zum Anschluss des Atemkreislaufs (190) des Patienten und einer Kopplungsvorrichtung (300) aufweist, die mit den Luftstromkreisläufen der Belüftungsanordnungen (100, 200) und mit der Verbindungsanordnung (303) des Geräts (1) verbunden ist, wobei der Atemkreislauf des Patienten daher in der Lage ist, dass er über die Kopplungsvorrichtung (300) an die Beatmungsanordnungen (100, 200) angeschlossen zu werden; wobei die Kopplungsvorrichtung (300) pneumatische oder pneumo-elektrische Verbindungsstellen zum Koppeln der Beatmungsanordnungen (100, 200) und Kopplungsventile (313, 314; 323, 324) an den Luftstromverbindungsstellen bezüglich jeder Beatmungsanordnung umfasst, wobei jede Beatmungsanordnung weiter eine Kopplungssteuerung (120, 220) aufweist, die in der Lage ist, die Kopplungsventile der Luftstromverbindungsstellen bezüglich jeder Beatmungsanordnung zu steuern, um einen automatischen Austausch der einen gegen die andere in den Betriebsarten zu ermöglichen:
- Hochleistungskopplung, bei der die zwei Beatmungsanordnungen in dem Gerät betrieben werden und alle zwei dem Patienten einen Luftstrom bereitstellen, und/oder
- Hochsicherheitskopplung, bei der der Luftstrom von einer der Anordnungen bereitgestellt wird, wobei die andere Anordnung in Bereitschaft und in der Lage ist, sofort von der ersten zu übernehmen im Falle der Feststellung einer Anomalie der Letzteren.

2. Gerät zur Atmungsunterstützung (1) für eine Person/einen Patienten, die/der unter Atembeschwerden leidet, nach Anspruch 1, **dadurch gekennzeichnet, dass** jede Beatmungsanordnung eine Steuereinheit (102, 202) aufweist, die in der Lage ist, die Kopplungsventile der Luftstromverbindungsstellen bezüglich jeder Beatmungsanordnung zu steuern.

3. Gerät zur Atmungsunterstützung nach Anspruch 1, **dadurch gekennzeichnet, dass** die Steuereinheit (102, 202) eine Mensch-Maschine-Schnittstelle und eine elektronische Steuerung (113, 213) umfasst, die in der Lage ist, den Luftgenerator zu steuern.

4. Gerät zur Atmungsunterstützung (1) für eine Person/einen Patienten, die/der unter Atembeschwerden leidet, nach Anspruch 2, **dadurch gekennzeichnet, dass** die Kopplungssteuerung ein Ventil aufweist, dessen Öffnen und/oder Schließen durch ein von der Steuereinheit (102, 202) erzeugtes Signal gesteuert wird, sodass der automatische Austausch von einer Beatmungsanordnung zur anderen entsprechend den Betriebsarten Hochleistungskopplung und/oder Hochsicherheitskopplung ermöglicht wird.

5. Gerät zur Atmungsunterstützung nach einem der vorstehenden Ansprüche, **dadurch gekennzeichnet, dass** jede Beatmungsanordnung Druck- und/oder Luftdurchflusssensoren aufweist.

6. Gerät zur Atmungsunterstützung nach Anspruch 1, **dadurch gekennzeichnet, dass** die Kopplungsvorrichtung (300) umfasst:
- ein erstes Kopplungsventil (313), das an der Verbindungsstelle des eingeatmeten Luftstroms bezüglich der ersten Beatmungsanordnung angeordnet ist, und ein zweites Kopplungsventil (323), das an dem eingeatmeten Luftstrom aus der zweiten Beatmungsanordnung (100) angeordnet ist,
- ein erstes Kopplungsventil (314), das an der Verbindungsstelle des ausgeatmeten Luftstroms der ersten Beatmungsanordnung angeordnet ist, und ein zweites Kopplungsventil (324), das an dem relativen ausgeatmeten Luftstrom der zweiten Beatmungsanordnung (200) angeordnet ist,
- wobei die Kopplungssteuerung (120, 220) jeder Beatmungsanordnung in der Lage ist, die Kopplungsventile (313,314; 323,324) zu steuern.

7. Gerät zur Atmungsunterstützung nach einem der vorstehenden Ansprüche, **dadurch gekennzeichnet, dass** die Kopplungsvorrichtung (300) in Form eines abnehmbaren Moduls vorliegt, das die Verbindungsanordnung (303) zum Anschluss des Atemkreislaufs umfasst.

8. Gerät zur Atmungsunterstützung nach einem der vorstehenden Ansprüche, **dadurch gekennzeichnet, dass** jede Beatmungsanordnung (100, 200) einen Sicherheitsdrucksensor (104, 204), einen proximalen Drucksensor (105, 205), einen internen Durchflusssensor (103, 203), einen proximalen Durchflusssensor (106, 206) umfasst, und dadurch, dass die Kopplungsvorrichtung (300) die Verbindung der zwei Beatmungsanordnungen untereinander sicherstellt, indem sie die folgenden Elemente verbindet:
- Verbindung der Sicherheitsdrucksensoren (104 und 204) der zwei Anordnungen (100 und 200),
- Verbindung der proximalen Drucksensoren (105 und 205) der zwei Anordnungen (100 und 200),
- Verbindung der internen Durchflusssensoren (103 und 203) der zwei Anordnungen (100 und 200),
- Verbindung der proximalen Durchflusssensoren (106 und 206) der zwei Anordnungen (100 und 200).

9. Gerät zur Atmungsunterstützung nach einem der vorstehenden Ansprüche, **dadurch gekennzeichnet, dass** jede Beatmungsanordnung (100, 200) in Form eines abnehmbaren Beatmungsblocks unabhängig von den anderen Beatmungsblöcken des Geräts vorliegt.

## Claims

1. Assisted breathing device (1) for a person/patient suffering from respiratory disorders comprising: - a first ventilation unit (100), a connection interface for the connecting of a breathing circuit (190) of the patient, said first ventilation unit including at least one air generator (101), at least one pressure and/or flow rate sensor and an air flow circuit connected to the connection interface, - at least one second ventilation unit (200) including at least one air generator, at least one pressure and/or flow rate sensor and an air flow circuit connected to said connection interface; **characterised in that** the connection interface of the device includes a connection unit (303) intended for the connecting of the breathing circuit (190) of the patient and a coupling device (300) connected to the air flow circuits of the ventilation units (100, 200) and to the connection unit (303) of the device (1), the breathing circuit of the patient being thus able to be connected to said ventilation units (100, 200) via the coupling device (300); said coupling device (300) comprising pneumatic or pneumo-electric connections for the coupling of the ventilation units (100, 200) and of the coupling valves (313, 314; 323, 324) on the air flow connections relative to each ventilation unit, each ventilation unit further including a coupling control (120, 220) capable of controlling the coupling valves of the air flow connections relative to each ventilation unit so as to make it possible for an automatic substitution of one with the other in the operating modes:
- high-performance coupling, wherein the two ventilation units are turned on in the device and both supply an air flow to the patient, and/or
- high-safety coupling, wherein the air flow is provided by one of the units, with the other unit being on standby and capable of instantly taking over from the first in case an anomaly of the latter is detected.

2. Assisted breathing device (1) for a person/patient suffering from respiratory disorders according to claim 1, **characterised in that** each ventilation unit includes a control unit (102, 202) capable of controlling the coupling valves of the air flow connections relative to each ventilation unit.

3. Assisted breathing device according to claim 1, **characterised in that** the control unit (102, 202) comprises a man-machine interface and an electronic control (113, 213) capable of controlling the air generator.

4. Assisted breathing device (1) for a person/patient suffering from respiratory disorders according to claim 2, **characterised in that** the coupling control includes a valve of which the opening and/or the closing are controlled by a signal generated by the control unit (102, 202) so as to make it possible for the automatic substitution of one ventilation unit with the other according to the high-performance coupling and/or high-safety coupling operating modes.

5. Assisted breathing device according to any one of the preceding claims, **characterised in that** each ventilation unit includes pressure and/or air flow sensors.

6. Assisted breathing device according to claim 1, **characterised in that** the coupling device (300) comprises:
- a first coupling valve (313) arranged on the connection of the flow of inhaled air relative to the first ventilation unit and a second coupling valve (323) arranged on the flow of inhaled air coming from the second ventilation unit (100),
- a first coupling valve (314) arranged on the connection of the exhaled air flow of the first ventilation unit and a second coupling valve (324) arranged on the exhaled air flow relative to the second ventilation unit (200),
- the coupling control (120, 220) of each ventilation unit being capable of controlling said coupling valves (313, 314; 323, 324).

7. Assisted breathing device according to any one of the preceding claims, **characterised in that**, the coupling device (300) has the form of a removable module comprising the connection unit (303) intended for the connecting of the breathing circuit.

8. Assisted breathing device according to any one of the preceding claims, **characterised in that** each ventilation unit (100, 200) comprises a safety pressure sensor (104, 204), a proximal pressure sensor (105, 205), an internal flow rate sensor (103, 203), a proximal flow rate sensor (106, 206) and **in that** the coupling device (300) provides the interconnection of the two ventilation units by connecting said following elements:
- Connection of the safety pressure sensors (104 and 204) of the two unit (100 and 200),
- Connection of the proximal pressure sensors (105 and 205) of the two units (100 and 200),
- Connection of the internal flow rate sensors (103 and 203) of the two units (100 and 200),
- Connection of the proximal flow rate sensors (106 and 206) of the two units (100 and 200).

9. Assisted breathing device according to any one of the preceding claims, **characterised in that** each ventilation unit (100, 200) has the form of a removable ventilation unit that is independent from the other ventilation units of the device.
